# EUROPÄISCHE PATENTSCHRIFT

(11) **EP 1 360 951 B1**
(45) Veröffentlichungstag und Bekanntmachung des Hinweises auf die Patenterteilung: **29.12.2004**
(21) Anmeldenummer: 03014843.1
(22) Anmeldetag: 22.01.1999
(51) Int. Cl.: A61F 2/52

(54) **Verfahren zum Aufbringen einer dauernd klebrig bleibenden Schicht auf die Innenseite einer Brustprothese**
Process for depositing a durably sticky layer upon the interior surface of a mammary prosthesis
Procédé pour déposer une couche durablement collante sur la surface intérieure d'une prothèse mammaire

(30) Priorität: 21.04.1998 DE 19817769
(43) Veröffentlichungstag der Anmeldung: 12.11.2003
(62) Teilanmeldung aus: 99101207.1
(73) Patentinhaber: AMOENA Medizin-Orthopädie-Technik GmbH, D-83064 Raubling (DE)
(72) Erfinder: Reitmaier, Paul, 83547 Babensham (DE); Esterer, Ulrike, 83352 Altenmarkt (DE); Stelter, Nils, 83101 Achenmühle (DE); Stuffer, Georg, 83126 Flintsbach (DE); Stuffer, Hans, 83131 Nussdorf (DE)
(74) Vertreter: Laufhütte, Dieter, Dr.-Ing.

(56) Entgegenhaltungen:
- EP-A- 0 542 119
- EP-A- 0 588 756
- DE-A- 2 802 375
- DE-A- 4 211 542
- DE-U- 9 204 743

## Beschreibung

Die Erfindung betrifft ein Verfahren zum Aufbringen einer dauernd klebrig bleibenden Schicht aus einer klebrig eingestellten additionsvernetzenden Zweikomponentensilikonkautschukmasse auf die Innenseite einer Brustprothese, bestehend aus einem schalenförmigen Körper aus einem weichelastischem Material, vorzugsweise aus einem Körper aus einer weich elastisch eingestellten additionsvernetzenden Zweikomponentensilikonkautschukmasse, der in Kunststoffolien eingeschweißt ist, die dessen Außenseite und dessen Innenseite abdecken, und der in einer Form unter Zuführung von Wärme ausgehärtet ist.

Aus EP 0 542 119 B1 und DE 42 11 542 A sind Verfahren dieser Art bekannt, nach denen einmal eine klebrig eingestellte additionsvernetzende Zweikomponentensilikonkautschukmasse zur Bildung der dauernd klebrig bleibenden Schicht mit einer Tragfolie in eine Form eingebracht und in dieser ausgehärtet wird und anschließend die Tragfolie mit der die Prothese einhüllenden Folie auf ihrer Rückseite in ihrem Randbereich verklebt oder verschweißt wird und zum anderen eine klebrig eingestellte additionsvemetzende Zweikomponentensilikonkautschukmasse zur Bildung der dauernd klebrig bleibenden Schicht mit einer Tragfolie in eine Form eingebracht und in dieser ausgehärtet wird und auf die andere Seite der Tragfolie ein Schmelzkleber aufgebracht wird und anschließend die Tragfolie auf die die Rückseite des Körpers einhüllende Folie aufgebracht und während des Aushärtungsprozesses des Körpers durch Aufschmelzen und Erhärten des Schmelzklebers mit der Folie verbunden wird.

Ferner ist aus EP 0 588 756 A ein Haftelement mit beidseitigen durch Trennfolien geschützten Klebeschichten bekannt.

Aufgabe der Erfindung ist es, weitere Verfahren vorzuschlagen, nach denen sich in einfacher und vorteilhafter Weise dauernd klebrig bleibende Schichten mit Brustprothesen der eingangs angegebenen Art verbinden lassen.

Die Aufgabe der Erfindung wird durch Kombination der Merkmale des Anspruchs 1 dadurch gelöst, daß eine Klebeschicht in einer separaten Form mit einer mittleren aussteifenden Einlage hergestellt, beidseits durch Trennfolien geschützt und erst vor dem Anlegen der Prothese einseitig mit der Prothesenrückseite verklebt wird. Die Trägerin zieht vor dem Anlegen der Prothese die Trennfolie einer Seite ab und verklebt die in sich stabile Klebeschicht mit der Rückseite der Prothese. Sodann zieht sie die zweite Trennschicht ab und kann die Prothese anlegen.

Zweckmäßigerweise besteht die Einlage aus einer Gewebeeinlage. Dies hat den Vorteil, daß der Zweikomponentensilikonkautschuk bei der Füllung der Form zur Herstellung der Klebeschicht durch die Einlage hindurchtreten und sich beidseits von dieser ausbreiten kann.

Die Prothesenrückseite kann mit einem Haftvermittler versehen sein, der bleibende Verbindung mit der aufgebrachten Klebeschicht verwirkt. Nach dieser Ausgestaltung ist sichergestellt, daß die Klebeschicht beim Abnehmen der Prothese nicht am Körper, sondern an der Prothesenrückseite haftet.

Ausführungsbeispiele der Erfindung werden nachstehend anhand der Zeichnung näher erläutert. In dieser zeigt
- Fig. 1: einen Querschnitt durch eine in einer nicht dargestellten Form liegende Brustprothese, die durch einen Formdeckel abgedeckt ist, der auf seiner der Prothese zugewandten Seite mit einem der aufzubringenden Klebeschicht entsprechenden Kanal versehen ist,
- Fig. 2: eine der Fig. 1 entsprechende Darstellung, bei der zwischen dem Formdeckel und der Prothesenrückseite ein flexibler Streifen mit einer Ausnehmung eingelegt ist, die mit dem Klebestreifen gefüllt ist, und
- Fig. 3: eine der Fig. 1 entsprechende Darstellung, bei der auf die Prothesenrückseite eine abziehbare Folie aufgebracht ist, zwischen der und der Prothesenrückseite ein der Form der Klebeschicht entsprechender Kanal eingebracht ist, der mit dem die Klebeschicht bildenden Zweikomponentensilikonkautschuk gefüllt ist.

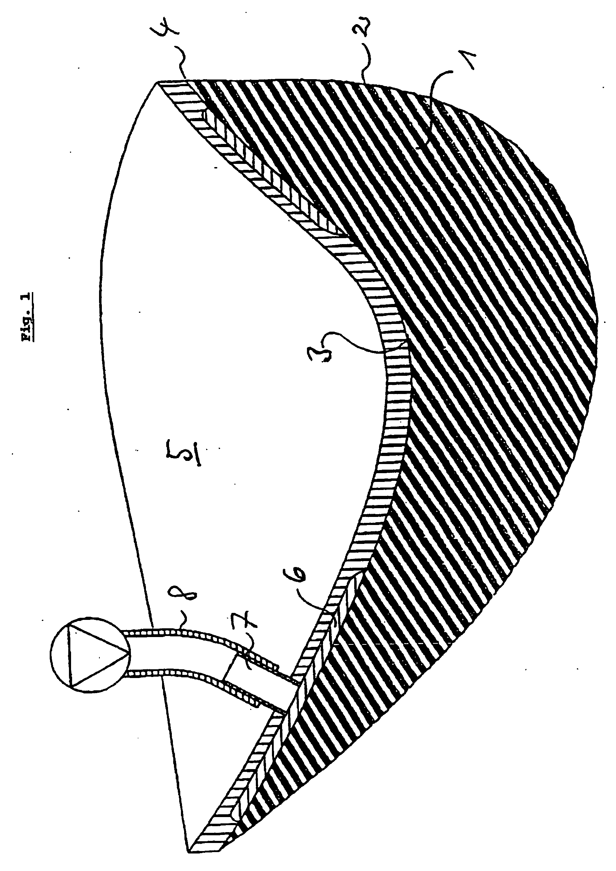

Nach dem Ausführungsbeispiel der Fig. 1 ist in eine nicht dargestellte Unterschale einer Form eine mit einem additionsvernetzenden Zweikomponentensilikonkautschuk 1 gefüllte Hülle eingelegt, die aus einer die Prothesenvorderseite abdeckenden Folie 2 und einer die Prothesenrückseite abdeckenden Folie 3 besteht, die längs ihres umlaufenden Randes 4 miteinander verschweißt sind. Die Prothesenhülle wird vor der Befüllung aus flach aufeinanderliegenden PU-Folien hergestellt. Der Zweikomponentensilikonkautschuk wird durch eine Randöffnung eingefüllt, so daß die Prothesenhülle aufgrund der Elastizität des Folienwerkstoffs die Form der späteren Brustprothese annimmt.

Der Formdeckel 5 weist auf seiner der Prothesenrückseite zugewandten Seite eine flache einen Kanal bildende Ausnehmung 6 auf, deren Form eine Negativform der auf die Prothesenrückseite aufzubringenden Klebeschicht ist. In den Kanal mündet ein in eine Bohrung des Formdeckels eingesetzter Stutzen 7, an den ein Schlauch 8 angeschlossen ist, über den in die rinnenartige Ausnehmung 6 nach dem Schließen des Formdeckels 5 der die Klebeschicht bildende Zweikomponentensilikonkautschuk über eine Pumpe eingefüllt wird.

Nach dem Schließen der Form und dem Einbringen des die Klebeschicht bildenden Zweikomponentensilikonkautschuks wird diese zusammen mit dem Prothesenkörper in üblicher Weise in einem Ofen ausgehärtet.

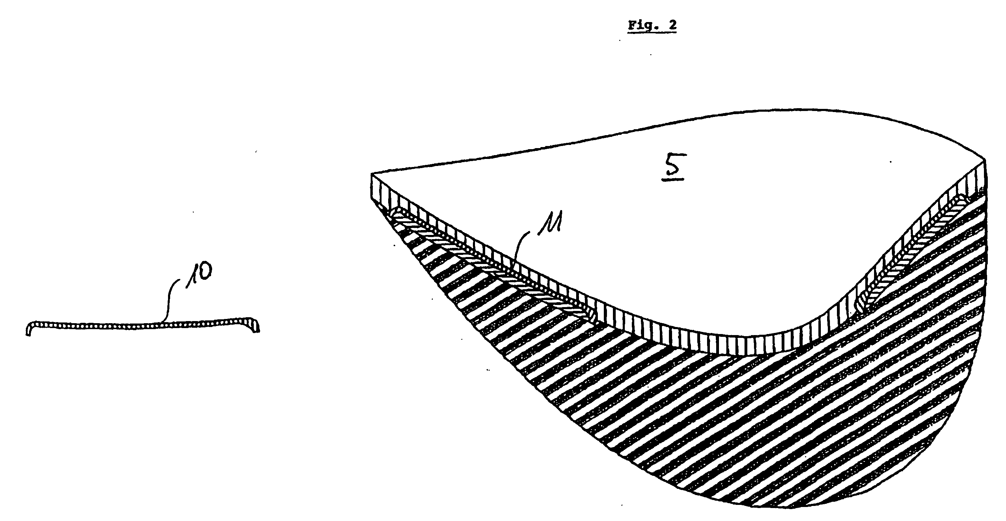

Bei dem Ausführungsbeispiel nach Fig. 2 wird zunächst in einer flachen Schale aus einem elastischen und/oder plastischen thermoplastischen Werkstoff mit einer rinnenförmigen Ausnehmung, die der Form der späteren Klebeschicht entspricht, in einem besonderen Herstellungsprozeß eine dauernd klebrig bleibende Schicht aus einer Zweikomponentensilikonkautschukmasse hergestellt. Die Schale 10 mit der in dieser ausgehärteten Klebeschicht 11 wird sodann in eine der Schalenform angepaßte Aussparung des Formdeckels 5 eingelegt und mit dem so vorbereiteten Formdeckel wird sodann die Form geschlossen. Die dauernd klebrig bleibende Schicht wird sodann nochmals zusammen mit dem Prothesenkörper in einem Ofen ausgehärtet, so daß sich die Klebeschicht mit der die Prothesenrückseite abdeckenden Folie verbindet. Nach dem Öffnen der Form wird die flache Schale 10 von der Klebeschicht abgezogen, so daß sie erneut verwendet werden kann. Die so hergestellte und mit der Prothesenrückseite verbundene Klebeschicht wird dann durch eine Trennfolie abgedeckt.

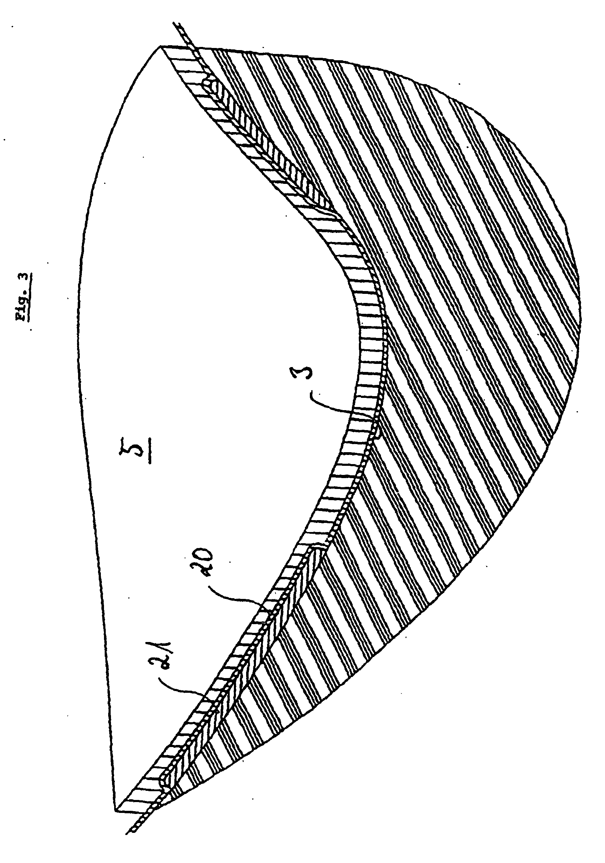

Bei dem Ausführungsbeispiel nach Fig. 3 wird auf die die Prothesenrückseite bildende Folie 3 eine abziehbare Trennfolie 20 aufgebracht, die mit der Folie 3 in der Weise verbunden ist, daß zwischen beiden ein Kanal 21 gebildet ist, der der Form der aufzubringenden Klebeschicht entspricht. Dieser Kanal 21 wird vor dem Schließen der Form mit dem Formdeckel 5 mit einer die Klebeschicht bildenden Zweikomponentensilikonkautschukmasse gefüllt. In den Formdeckel ist ein Kanal eingearbeitet, der der Form der Klebeschicht entspricht. Nachdem der Kanal mit der die Klebeschicht bildenden Zweikomponentensilikonkautschukmasse gefüllt ist, wird nach dem Schließen der Form diese mit dem Prothesenkörper ausgehärtet. Der Form kann sodann die Prothese entnommen werden, wobei die Klebeschicht durch die Trennfolie 21 geschützt ist, die von der Trägerin erst vor dem Anlegen abgezogen wird.

## Patentansprüche

1. Verfahren zum Aufbringen einer dauernd klebrig bleibenden Schicht aus einer klebrig eingestellten additionsvernetzenden Zweikomponentensilikonkautschukmasse auf die Innenseite einer Brustprothese, bestehend aus einem schalenförmigen Körper aus einem weichelastischem Material, vorzugsweise aus einem Körper aus einer weichelastisch eingestellten additionsvemetzenden Zweikomponentensilikonkautschukmasse, der in Kunststoffolien eingeschweißt ist, die dessen Außenseite und dessen Innenseite abdecken, und der in einer Form unter Zuführung von Wärme ausgehärtet ist,
**dadurch gekennzeichnet,**
**daß** eine Klebeschicht in einer separaten Form mit einer mittleren aussteifenden Einlage hergestellt, beidseitig durch Trennfolien geschützt und erst vor dem Anlegen einseitig mit der Prothesenrückseite verklebt wird.

2. Verfahren nach Anspruch 1, **dadurch gekennzeichnet, daß** die Einlage aus einer Gewebeeinlage besteht.

3. Verfahren nach Anspruch 1 oder 2, **dadurch gekennzeichnet, dass** die Prothesenrückseite mit einem Haftvermittler versehen wird, der eine bleibende Verbindung mit der aufgebrachten Klebeschicht bewirkt.

## Claims

1. Method for the application of a layer remaining permanently adhesive made from an adhesively formulated addition-crosslinking two-component silicone rubber mass to the inside of a breast prosthesis consisting of a shell-shaped body made from a flexible material, preferably of a body made from a flexibly formulated addition-crosslinking two-component silicone rubber mass, which body is welded in plastic films which cover its outside and its inside, and which is cured in a mould while heat is supplied,
**characterized**
**in that** an adhesive layer is produced in a separate mould with a central stiffening insert, protected on both sides by release films and not bonded one-sidedly to the reverse of the prosthesis until before placement.

2. Method according to Claim 1, **characterized in that** the insert is composed of a woven insert.

3. Method according to Claim 1 or 2, **characterized in that** the reverse of the prosthesis is provided with an adhesion promoter which effects a permanent connection with the applied adhesive layer.

## Revendications

1. Procédé pour appliquer une couche durablement collante en une masse de caoutchouc au silicone à deux composants réticulant par addition réglée pour être collante sur le côté intérieur d'une prothèse mammaire, constituée d'un corps en forme de coque en un matériau élastique mou, de préférence en un corps d'une masse de caoutchouc au silicone à deux composants réticulant par addition, réglée pour être molle et élastique, qui est soudé dans des feuilles de matériau synthétique qui recouvrent son côté extérieur et son côté intérieur et qui est durcie dans un moule sous amenée de chaleur,
**caractérisé en ce qu'**une couche de colle est fabriquée dans un moule séparé avec un insert médian de renforcement, est protégée des deux côtés par des feuilles de séparation et est assemblée par collage seulement avant la mise en place sur un côté avec le côté arrière de la prothèse.

2. Procédé selon la revendication 1, **caractérisé en ce que** l'insert est constitué d'un insert de tissu.

3. Procédé selon la revendication 1 ou 2, **caractérisé en ce que** le côté arrière de la prothèse est pourvu d'un agent adhésif qui provoque une liaison durable avec la couche de colle appliquée.
